**Europäisches Patentamt**

**European Patent Office**

**Office européen des brevets**

(11) Veröffentlichungsnummer: **0 340 576**
**A2**

## (12) EUROPÄISCHE PATENTANMELDUNG

(21) Anmeldenummer: 89107317.3

(22) Anmeldetag: 22.04.89

(51) Int. Cl.⁴: **C07C 43/04** , **C07C 41/09** ,
**C07C 41/42**

(30) Priorität: 04.05.88 EP 88107118

(43) Veröffentlichungstag der Anmeldung:
08.11.89 Patentblatt 89/45

(84) Benannte Vertragsstaaten:
AT BE CH DE ES FR GB GR IT LI LU NL SE

(71) Anmelder: **Union Rheinische Braunkohlen
Kraftstoff Aktiengesellschaft
Ludwigshafener Strasse o. Nr. Postfach 1663
D-5047 Wesseling(DE)**

(72) Erfinder: **Dornhagen, Horst, Dr.
Auf dem Radacker 21
D-5047 Wesseling(DE)**
Erfinder: **Hammer, Hartmut, Dr.
Leyboldstrasse 21
D-5000 Köln 1(DE)**
Erfinder: **Meisenburg, Ewald
Höhenring 60
D-5357 Swisttal-Heimerzheim(DE)**
Erfinder: **Schmid, Horst
Am Schmettenstück 29
D-5047 Wesseling(DE)**

(54) Verfahren zur Gewinnung von reinem Dimethylether (DME).

(57) Die Erfindung betrifft ein Verfahren zur Gewinnung von reinem Dimethylether durch Zuführung des Dehydratisierungsprodukts aus einem Dimethylether-Synthesereaktor in eine Destillationskolonne zur Gewinnung von reinem Dimethylether an bestimmten Böden dieser Kolonne und Entnahme des reinen Dimethylethers und einer Verunreinigungen enthaltenden Fraktion an bestimmten Böden der gleichen Kolonne, wobei zusätzlich zum Dehydratisierungsprodukt eine Waschflüssigkeit und Basen eingesetzt werden können.

## Verfahren zur Gewinnung von reinem Dimethylether (DME)

Die Erfindung betrifft ein Verfahren zur Gewinnung von reinem Dimethylether durch Zuführung des Dehydratisierungsprodukts aus einem Dimethylether-Synthesereaktor in eine Destillationskolonne zur Gewinnung von reinem Dimethylether an bestimmten Böden dieser Kolonne und Entnahme des reinen Dimethylethers und einer Verunreinigungen enthaltenden Fraktion an bestimmten Böden der gleichen Kolonne, wobei zusätzlich zum Dehydratisierungsprodukt eine Waschflüssigkeit und Basen eingesetzt werden können.

Bis zur Entwicklung der Methanol-Niederdruck-Synthese-Verfahren wurde Dimethylether als Nebenprodukt der Methanol-Hochdruck-Synthese in einer Menge von etwa 2 - 5 Gew.-% erhalten, bezogen auf die Gesamtmenge des Synthesereaktor-Ausgangsprodukts und bei der Gewinnung von Reinmethanol destillativ aus dem weitere Verunreinigungen enthaltenden Vorlauf abgetrennt.

Nach Einführung der Niederdruck-Methanolsynthese-Verfahren, bei denen keine nennenswerten Mengen an Dimethylether anfallen, wurden Syntheseverfahren zur Herstellung von Dimethylether durch katalytische Dehydratisierung von Methanol entwickelt.

Zahlreiche Verfahren sind in der Patentliteratur beschrieben. So lassen sich nach DE-PS 680 328 aliphatische Ether durch Erhitzen von Alkoholen in Gegenwart von Zinkchlorid erzeugen.

Weitere geeignete Katalysatoren zur Herstellung von Ethern aus Alkoholen sind gemäß GB-PS 332 756, GB-PS 350 010, GB-PS 403 402, US-PS 1 873 537 und FR-PS 701 335 Eisenchlorid, Kupfersulfat, Zinnchlorid, Manganchlorid, Aluminiumchlorid und -sulfat, Chromsulfat, Alaune, Thorium-Verbindungen, Aluminiumoxid, Titanoxid, Bariumoxid, Silicagel oder Aluminiumphosphat. In "Industrial and Engineering Chemistry", Vol. 41, No. 12, S. 2928, 1949 wird die Verwendung von Bauxiten mit $SiO_2$-Gehalten von 4,40 - 13,99 Gew.-% beschrieben.

In US-PS 3 036 134 wird ein Aluminiumsilikat-Katalysator offenbart, zur Herstellung von Dimethylether aus Methanol, der ein $Al_2O_3$:$SiO_2$-Verhältnis von 1 Teil zu 1,35 bis 0,3 Teilen aufweist.

Auch die Synthese von Dimethylether direkt aus Synthesegas (CO + $H_2$) ist beschrieben worden (DE-PS 23 62 944, DE-PS 27 57 788 und DE-PS 32 20 547).

Als technisch wichtigste Katalysatoren haben sich gemäß DE-PS 28 18 831, DE-OS 32 01 155, EP-A 0 099 676 und EP-A 0 124 078 insbesondere Aluminiumoxid- und Aluminiumsilikat-Katalysatoren mit und ohne Dotierung durchgesetzt.

In DE-PS 28 18 831 wird ein Katalysator zur Herstellung von Dimethylether offenbart, der jedes beliebige Aluminiumoxid-Grundmaterial mit genügend großer Oberfläche enthalten kann, in dem zusätzlich seltene Erden mit 1 - 30 Gew.-% $SE_2O_3$ vorhanden sind.

In EP-A 0 099 676 ist ein Katalysator beschrieben, der 1 - 20 Gew.-% $SiO_2$, vorzugsweise 1 - 10 Gew.-% $SiO_2$ und noch bevorzugter 6 Gew.-% $SiO_2$ enthält.

Der hierbei anfallende rohe Dimethylether enthält das Reaktionswasser, nicht umgesetztes Methanol sowie kleine Mengen an Verunreinigungen, wie beispielsweise Methylformiat, Kohlenwasserstoffe, Amine und Sulfide, Carbonsäuren, Ester, Amide, Azetale u.a.

In der europäischen Patentanmeldung EP-A 0 270 852 wird ein Verfahren beschrieben, gemäß dem Dimethylether aus Methanol in Gegenwart γ-$Al_2O_3$-Katalysators mit sehr geringem $SiO_2$-Gehalt erzeugt wird.

In diesen Synthese-Anlagen wird der rohe Dimethylether in zwei hintereinander geschalteten Destillationskolonnen aufgearbeitet, wobei in der ersten, unter Druck betriebenen Kolonne Dimethylether gewonnen wird und in der zweiten Kolonne nicht umgesetztes Methanol zurückgewonnen wird.

So wird in EP-A 0 124 078 ein Verfahren beschrieben, wonach in einer ersten unter Druck stehenden Kolonne, Dimethylether im Seitenabzug entnommen wird, während in einer zweiten unter niedrigerem Druck arbeitenden Kolonne über Kopf die zwischen Dimethylether und Methanol siedenden Verunreinigungen abgenommen werden. Methanol wird ebenfalls aus der 2. Kolonne im Seitenabzug entnommen.

Als Katalysator können $Al_2O_3$, $SiO_2$ Aluminiumsilikat und bevorzugt γ-Aluminiumoxid eingesetzt werden.

Obgleich bei diesem Verfahren ein gegenüber dem Stand der Technik reinerer Dimethylether gewonnen werden kann, hat es den erheblichen wirtschaftlichen Nachteil, daß nicht nur die erste, sondern auch die 2. Destillationskolonne mit hoher Bodenzahl ausgestattet sein müssen, so daß sowohl hohe Investitionskosten als auch insbesondere hohe Betriebskosten entstehen und daß ferner die Gefahr besteht, daß die zwischen Dimethylether und Methanol siedenden Verunreinigungen nicht vollständig in die 2. Kolonne gelangen, sondern nach Anreicherung in der 1. Kolonne mit dem Dimethylether abgezogen werden, so daß keine Geruchsfreiheit vorliegt.

In der oben bereits genannten EP-A 0 270 852 wird ein destillatives Reinigungsverfahren zur Gewinnung von hochreinem, geruchsfreiem Dimethylether beschrieben, wobei die zwischen Dimethylether und Methanol siedenden Verunreinigungen in der gleichen Kolonne in der auch der reine Dimethylether destillativ gewonnen wird, an bestimmten Böden im Seitenabzug abgetrennt werden.

Da Dimethylether als Treibmittel für Aerosolsprays zunehmend an Bedeutung gewinnt, werden für bestimmte Anwendungen sehr hohe Reinheitsanforderungen an dieses Produkt gestellt. So dürfen insbesondere für die kosmetische, human- und Haushalts-Anwendung keine irritierenden Substanzen im Dimethylether enthalten sein. Ferner muß der Dimethylether hierbei frei bzw. weitgehend frei von Geruchsstoffen sein.

Es bestand daher die Aufgabe, die Gewinnung von hochreinem Dimethylether weiter zu verbessern.

Die Lösung dieser Aufgabe ist der Anmelderin in hervorragender Weise durch das erfindungsgemäße Verfahren gelungen, nämlich durch die Gewinnung von reinem Dimethylether durch katalytische Dehydratisierung von Methanol bei einer Temperatur von 140 - 500 °C und einem Druck von 1 - 50 bar und destillative Aufarbeitung des Dehydratisierungsprodukts, durch Einsatz des gasförmigen und/oder flüssigen Dehydratisierungsprodukts in die Kolonne zur Gewinnung von reinem Dimethylether mindestens 1 Boden, bevorzugt mindestens 5 Böden und besonders bevorzugt mindestens 10 Böden oberhalb des Kolonnensumpfes, dadurch gekennzeichnet

a, daß (eine) Verunreinigungen enthaltende flüssige und/oder gasförmige Fraktion(en), an einem oder mehreren Böden abgezogen wird (werden), wobei
die Verunreinigungen enthaltende(n) Fraktion(en) um mindestens 3, bevorzugt mindestens 5 Böden unterhalb des untersten Abzugsbodens für reinen Dimethylether abgezogen wird (werden) bis mindestens 5 Böden bevorzugt mindestens 10 Böden oberhalb des Sumpfes.

b, daß der Boden, oder im Falle mehrerer Abzugsböden der unterste Boden an dem der reine Dimethylether abgezogen wird
bei nur einem Zuführungsboden um mindestens 8 Böden, bevorzugt mindestens 10 Böden und besonders bevorzugt um mindestens 12 Böden oberhalb des Bodens liegt, an dem das Einsatzprodukt zugeführt wird
bei mehreren Zuführungsböden bei einer am obersten Zuführungsboden zugeführten Menge von > 0 - < 100 % der Gesamtmenge, um mindestens 1 Boden bis mindestens 8 Böden oberhalb des obersten Zuführungsbodens liegt, wobei mit steigender zugeführter Menge der Bodenabstand zunimmt.

Die Bestimmung geruchsbelästigender Substanzen erfolgt noch immer überwiegend empirisch, wobei direkte sensorische Messungen durch ein geübtes Team erfolgen. So wurde beispielsweise die Belästigungsschwelle "BS" für $H_2S$ durch ein Testkollektiv von 150 Personen auf 45 $\mu g/m^3$ ermittelt (Schriftenreihe der Landesanstalt für Immissionsschutz des Landes Nordrhein-Westfalen, Heft 49 (1979) S.77).

In den Fällen, in denen die Geruchsschwelle im durch Instrumente messbaren Bereich liegt, können Geruchsschwellen bzw. Belästigungsschwellen auch durch Meßmethoden wie Gaschromatographie, elektrische Leitfähigkeit, Photometrie oder Fluoreszenzmessung bestimmt werden ("Erdöl und Kohle-Ergas-Petrochemie", Bd. 32, Heft 2, Febr. 1975, S.86).

Die Geruchsbestimmungen im Falle der vorliegenden Erfindung beruhen auf der sensorischen Methode.

Sowohl Rohmethanol und Reinmethanol aus einer Methanolsyntheseanlage als auch katalytisch aus Methanol hergestellter Dimethylether enthalten wie bereits oben ausgeführt, zahlreiche Verunreinigungen, die teilweise sehr geruchsintensiv sind. Während in Methanol-Hochdrucksyntheseanlagen ein Rohmethanol anfällt, in dem im allgemeinen 2 - 3, jedoch bis zu 5 Gew.-% Dimethylether enthalten sein können, werden bei Dimethylethersynthesen aus Roh- oder Reinmethanol je nach Fahrweise im allgemeinen 20 bis 80 Gew.-% Dimethylether aus dem eingesetzten Methanol am Reaktorausgang erhalten. Zusätzlich sind im rohen Dimethylether die oben genannten Verunreinigungen, Reaktionswasser und nicht umgesetztes Methanol enthalten.

Da die Siedepunkte der Verunreinigungen, wie beispielsweise Methylamin (Kp -7,55 °C/719), von Dimethylamin (Kp 6,9 °C), Trimethylamin (Kp -3 °C), Dimethylsulfid (Kp 37,3 °C), Methylmercaptan (Kp 5,8 °C), Ameisensäure (Kp 100,75 °C), Ameisensäuremethylester (Kp 31,5 °C), Formaldehyd (Kp -21 °C), Formaldehyddimethylacetal (Kp 45,5 °C), oder Essigsäuremethylester (Kp 56,95 °C) sowie ihre Löslichkeiten und Dampfdrucke im Produktgemisch sehr unterschiedlich sind und die subjektiv wahrgenommene Geruchsintensität der einzelnen Verunreinigungen ebenfalls sehr unterschiedlich ist und zusätzlich eine Reihe azeotroper Gemische gebildet werden, wird die Lösung der Aufgabe, hochreinen Dimethylether in hohen Ausbeuten nach einem besonders wirtschaftlichen Verfahren zu erzeugen sehr erschwert.

Die Anmelderin hat daher in mehrjährigen Untersuchungen durch zahlreiche Testreihen in Labor, Technikum und technischer Anlage überraschend gefunden, daß hochreiner Dimethylether praktisch quantitativ aus dem Dehydratisierungsgemisch (Rohprodukt aus der Synthese) durch die erfindungsgemäße destillative Reinigung erhalten werden kann.

In Figur 1 ist beispielhaft eine Anlage zur Erzeugung und Reinigung von Dimethylether dargestellt.

In Figur 2 ist beispielhaft eine Destillationskolonne für die Gewinnung von reinem Dimethylether dargestellt.

In Figur 3 ist beispielhaft diese Kolonne mit einem Vorlauf-Seitenstripper dargestellt.

In Figur 4 ist die Kombination von 2 Dimethylether-Kolonnen dargestellt.

Als in der Erfindung besonders geeignete Katalysatoren haben sich von der gleichen Anmelderin in der EP-A 0 270 852 offenbarte Katalysatoren vom $\gamma$-$Al_2O_3$-Typ erwiesen.
Dieser enthält nur sehr geringe Mengen $SiO_2$. Der $SiO_2$-Gehalt ist > 0 bis < 1 Gew.-%. Bevorzugt ist eine Menge an $SiO_2$ von 0,001 bis 0,5 Gew.-% und besonders bevorzugt von 0,001 bis 0,2 Gew.-% $SiO_2$.

Obgleich mit dem erfindungsgemäßen Verfahren diskontinuierlich gearbeitet werden kann, ist die kontinuierliche Fahrweise bevorzugt.

Die Untersuchungen der Anmelderin haben zu dem Ergebnis geführt, daß zur Lösung der Aufgabe, auf besonders wirtschaftliche Weise hochreinen geruchsfreien Dimethylether mit nahezu quantitativer Ausbeute gewinnen zu können, die Zuführung des Dehydratisierungsprodukts in die Destillationskolonne zur Gewinnung reinen Dimethylethers flüssig und oder gasförmig an einem oder mehreren Böden erfolgen kann der (die) mindestens 1 Boden, bevorzugt 5 Böden und besonders bevorzugt 10 Böden oberhalb des Kolonnensumpfes liegen muß (müssen).
Ferner liegt erfindungsgemäß der Abzugsboden oder im Falle mehrerer Abzugsböden der unterste Boden an dem der reine Dimethylether abgezogen wird
bei nur einem Zuführungsboden um mindestens 8 Böden, bevorzugt mindestens 10 Böden und besonders bevorzugt um mindestens 12 Böden oberhalb des Bodens an dem das Einsatzprodukt zugeführt wird
bei mehreren Zuführungsböden bei einer am obersten Zuführungsboden zugeführten Menge von > 0 - < 100 % der Gesamtmenge, um mindestens 1 Boden bis mindestens 8 Böden oberhalb des obersten Zuführungsbodens wobei mit steigender zugeführter Menge der Bodenabstand zunimmt.

Bevorzugte Abzugsböden für reinen DME sind der 15. und höhere Böden (näher am Kopf der Kolonne). Gegebenenfalls kann der reine Dimethylether auch als Kopfkondensat z.B. bei Einsatz einer Vorlaufkolonne abgezogen werden. Es können auch mehrere Böden zum Abzug von reinem Dimethylether genutzt werden. Der reine Dimethylether kann flüssig und/oder gasförmig abgezogen werden.

Die Untersuchungen der Anmelderin haben ferner ergeben, daß erfindungsgemäß (eine) Verunreinigungen enthaltende flüssige und/oder gasförmige Fraktion(en), an einem oder mehreren Böden abgezogen wird (werden), wobei die Verunreinigungen enthaltende(n) Fraktion(en) um mindestens 3, bevorzugt mindestens 5 Böden unterhalb des untersten Abzugsbodens für reinen Dimethylether abgezogen wird (werden), bis mindestens 5 Böden, bevorzugt mindestens 10 Böden oberhalb des Sumpfes.

Es ist also erfindungsgemäß möglich, Verunreinigungen enthaltende Fraktionen in diesem Bereich der Kolonne an einem oder mehreren Böden gasförmig und/oder flüssig abzuziehen, wobei naturgemäß, wie oben bereits angeführt, um so mehr Dimethylether in den abgezogenen Verunreinigungen enthalten ist, je näher der Boden für den Abzug der Verunreinigungen am Boden bzw. an den Böden für den Abzug des reinen Dimethylethers liegt. Es werden daher Verunreinigungen in einem Abstand von mindestens 3, bevorzugt mindestens 5 Böden vom Dimethyletherabzug bzw. bei mehreren vom untersten DME-Abzug entnommen. Es kann erfindungsgemäß verunreinigtes Produkt weiterhin bis mindestens 5 Böden, bevorzugt mindestens 10 Böden oberhalb des Sumpfes flüssig oder gasförmig abgezogen werden.

Erfindungsgemäß ist es auch möglich, die Menge der die Verunreinigungen enthaltenden Fraktion in weiten Grenzen zu variieren, so daß bei genügend großer Menge ein Produkt anfällt, das als sog. technische Dimethyletherqualität verwendet werden kann. Diese kann beispielsweise zur Herstellung von Dimethylsulfat dienen oder zum Einsatz in Sprays, in denen Geruchsfreiheit nicht gefordert wird. Auch zur Umsetzung zu Benzin kann diese Qualität eingesetzt werden.

Naturgemäß fällt bei diesem erfindungsgemäßen Fraktionieren weniger reiner, nicht geruchsfreier DME als technische Fraktion an.
Allgemein gilt, daß der Abzugboden für das die Verunreinigungen enthaltende Destillat umso näher am Abzugsboden für reinen DME liegen kann, je größer die Destillatmenge ist.

Erfindungsgemäß kann zusätzlich zum in dem Dehydratisierungsprodukt enthaltenen Methanol, eine Waschflüssigkeit eingesetzt werden, die bevorzugt Methanol ist.
Es kann sich hierbei um reines Methanol, um sog. Rohmethanol oder auch um andere Methanolsorten handeln.
Unter Rohmethanol wird Methanol verstanden, das direkt aus einer Methanolsynthese stammt. Hierbei kann es sich um eine Niederdruck-, Mitteldruck- oder Hochdruck-Methanolsynthese handeln, während reines Methanol durch Destillation gereinigtes Methanol ist, das den nationalen und internationalen Spezifikationen entspricht. Eine andere Methanolsorte kann beispielsweise ein in einer Vorlaufkolonne abgetopptes Rohmethanol sein, aus dem Gase wie $CH_4$, $CO_2$, $N_2$ und sonstige leichte Komponenten entfernt worden sind.

Die Waschflüssigkeit kann jedoch auch Wasser oder Methanol/Wasser-Gemisch oder auch Dimethylether im Gemisch mit Wasser und/oder Methanol sein.

Die Waschflüssigkeit kann, bevor sie in die Dimethylether-Destillationskolonne gelangt, dem Dehydratisierungsprodukt zugemischt werden, so daß das Gemisch auf den bzw. die Einspritzböden gelangt.

Grundsätzlich kann auch das Einsatzprodukt (Dehydratisierungsproduktgemisch) zumindest teilweise mit oder ohne Waschflüssigkeit gasförmig in die Kolonne eingesetzt werden.

Es kann auch eine Fahrweise gewählt werden, bei der die Waschflüssigkeit getrennt bzw. zumindest teilweise getrennt auf den gleichen Einspritzboden bzw. die gleichen Einspritzböden geleitet werden, auf den bzw. die auch das Dehydratisierungsprodukt geleitet wird.

Eine weitere erfindungsgemäße Fahrweise ist, daß die Waschflüssigkeit an anderen Böden als das Dehydratisierungsprodukt zugeführt wird. Bevorzugt wird die Waschflüssigkeit auf einem Zuführungsboden oder unterhalb des untersten Zuführungsbodens für das Dehydratisierungsprodukt eingesetzt.

Erfindungsgemäß ist es auch möglich das heiße, aus dem Dimethylether-Synthesereaktor austretende Produkt in einer vor der Destillationskolonne befindlichen Waschvorrichtung zumindest teilweise mit Waschflüssigkeit zu waschen. Während das gewaschene gasförmige Dehydratisierungsprodukt zur Destillationskolonne strömt, wird die Verunreinigungen enthaltende Waschflüssigkeit abgezogen und kann dem unteren Teil der Destillationskolonne zugeführt werden.

Der Waschflüssigkeitszusatz hat die vorteilhafte Wirkung eines zusätzlichen Reinigungseffektes bezüglich des zu gewinnenden hochreinen Dimethylethers. Die Menge der zugesetzten Waschflüssigkeit kann daher in sehr weiten Grenzen von > 0 %, bezogen auf die Menge des eingesetzten Dehydratisierungsproduktes, bis zu einem mehrfachen dieser Menge variieren. Maßgebend ist in erster Linie die Qualität des zu gewinnenden Dimethylethers.

Sollten Verunreinigungen bzw. störender Geruch in diesem auftreten, z.B. aufgrund einer schlechten Qualität des in die Dimethylethersynthese eingesetzten Methanols, so sollte die Menge der zugesetzten Waschflüssigkeit variiert werden. Ein weiteres Mengenkriterium ist die zwangsweise anfallende Produktionsmenge an Rohmethanol im Falle der Integration der Dimethyletherdestillation in eine Rohmethanoldestillation.

In diesem Fall kann je nach Produktionskapazität der Methanolsyntheseanlage und der Dimethylethersyntheseanlage bei gemeinsamer Destillation des Rohmethanols und des Dehydratisierungsprodukts aus der Dimethylethersyntheseanlage in einer Kolonne gemäß vorliegender Erfindung, in der an den genannten Böden der reine Dimethylether abgezogen wird, das Mengenverhältnis Rohmethanol zu Dehydratisierungsprodukt sehr stark variieren.

Dies wird für den Fachmann deutlich, wenn man davon ausgeht, daß Methanolsyntheseanlagen meist eine Jahreskapazität von 100.000 t bis 600.000 t besitzen, während Jahresproduktions-Kapazitäten von Dimethylethersyntheseanlagen etwa zwischen 5.000 t bis 100.000 t liegen. Beispielsweise kann das Verhältnis bei einer Dimethyletheranlage einer Kapazität von 5.000 t/a und einer Methanolkapazität von 500.000 t/a bei 1:100 liegen. Andererseits kann die Waschflüssigkeit nur ein Bruchteil der Menge an Dehydratisierungsprodukt sein, wie z.B. 1/100, 1/20, 1/10 oder 1/5. Gleiches gilt für den Einsatz der anderen Waschflüssigkeiten.

Es kann weiterhin von Vorteil sein, im Sumpf der Dimethylether-Destillationskolonne, insbesondere bei Einsatz von Rohmethanol, einen bestimmten pH-Wert von mindestens 7 einzustellen, bevorzugt pH > 7 und besonders bevorzugt pH > 8, durch Zugabe einer Base, insbesondere von NaOH, KOH und anderen Alkali- und Erdalkalibasen oder von Basengemischen. Die Zuführung kann grundsätzlich an einer beliebigen Stelle erfolgen, innerhalb und außerhalb der Dimethyletherkolonne, bevorzugt an dem Boden unterhalb des Bodens an dem das die Verunreinigungen enthaltende Destillat abgezogen wird.

Man kann erfindungsgemäß die Base auch in die Kolonne führende Leitung für das Dehydratisierungsprodukt bzw. für Dehydratisierungsprodukt und Methanol oder in die Methanolzuführungsleitung zugeben. Hierbei tritt die gewünschte Durchmischung bereits in der Leitung auf. Sie kann beispielsweise auch mit der Waschflüssigkeit zugeführt werden. Die aufgezeigten Zuführungsmöglichkeiten für die Base oder Basengemische sind beispielhaft, jedoch nicht als limitierend anzusehen.

Eine Base kann auch bereits in einer Vorlaufkolonne zugegeben werden.

Das Rückflußverhältnis in der Dimethyletherkolonne kann in Abhängigkeit von der Dimethylethermenge im Dehydratisierungsprodukt variiert werden.

Die umfangreichen Untersuchungen der Anmelderin haben zu dem überraschenden Ergebnis geführt, daß im Falle von nur einem Zuführungsboden ein hochreiner, geruchsfreier Dimethylether bereits dann erhalten wird, wenn der unterste Boden, an dem der reine Dimethylether abdestilliert wird um mindestens 8 Böden bevorzugt mindestens 10 und besonders bevorzugt mindestens 12 Böden höher liegt als der Zuführungsboden für das Dehydratisierungsprodukt, d.h. ist beispielsweise der Zuführungsboden der 26. Boden vom Kopf der Kolonne, so kann der reine Dimethylether bereits am 18. Boden oder höheren Böden abgenommen werden. Das die Verunreinigungen enthaltende Destillat kann dann bereits am 15. Boden oder tieferen Böden (vom Kopf der Kolonne) bis zum 5. Boden oberhalb des Sumpfes abgenommen werden.

Bevorzugt sollte das die Verunreinigungen enthaltende Destillat wenigstens 10 Böden oberhalb des Sumpfes und 5 Böden unterhalb des Dimethyletherabzugs abgezogen werden.

Dies bedeutet, daß das die Verunreinigungen enthaltende Destillat am 23. Boden abgezogen wird. Es kann jedoch erfindungsgemäß zwischen dem 23. und dem 10. Boden oberhalb des Sumpfes abgenommen werden, wobei es jedoch um so mehr Dimethylether enthält, je näher der Abzugsboden am Dimethyletherabzugsboden liegt.

Bei mehreren Zuführungsböden bei einer am obersten Zuführungsboden zugeführten Menge von > 0 - < 100 % von der Gesamtmenge, liegt der Boden oder im Falle mehrerer Abzugsböden der unterste Boden, an dem der reine Dimethylether abgezogen wird, um mindestens 1 Boden bis mindestens 8 Böden oberhalb des obersten Zuführungsbodens, wobei mit steigender zugeführter Menge der Bodenabstand zunimmt.

Die Untersuchungen der Anmelderin haben weiterhin zu dem überraschenden Ergebnis geführt, daß die Einstellung des pH's im Sumpf der Dimethyletherkolonne von großem Einfluß auf die Geruchskomponenten im Dimethylether ist.

So können auch bei Fahrweisen innerhalb des in den Ansprüchen angegebenen Schutzbereichs Grenzbereiche auftreten, in denen Geruchskomponenten in den reinen Dimethylether gelangen, beispielsweise bei einer Fahrweise mit sehr geringem Rückfluß und einem nur geringen Anteil Dimethylether im Einsatzprodukt; (denn innerhalb des beanspruchten Schutzbereichs ist das Rückflußverhältnis in Abhängigkeit von der Dimethyletherkonzentration so zu wählen, daß das gewünschte reine Produkt erhalten wird).

Auch im Falle des Abzugs einer sehr geringen, Verunreinigungen enthaltenden, Fraktion und einer nahezu quantitativen Abnahme von DME können Geruchskomponenten im letzteren auftreten. (Erfindungsgemäß ist eine solche Menge an die Verunreinigungen enthaltender Fraktion abzuziehen, daß diese die Geruchskomponenten vollständig bzw. nahezu vollständig aufnimmt. Diese Menge kann nach Anfahren der Kolonne durch Probenahme und Geruchsanalyse sehr schnell ermittelt werden.)

Ferner kann ein an Geruchskomponenten reiches Methanol, das in die DME-Synthese eingesetzt wird, zu Problemen führen oder das über Kopf oder Seitenabzug der der Dimethyletherkolonne nachgeschalteten Methanolkolonne, gewonnene Methanol, führt je nach enthaltenen Verunreinigungen bei der Rückführung in die DME-Synthese zu Geruchsproblemen.

Durch Einstellung des erfindungsgemäßen pH's im Sumpf der DME-Kolonne läßt sich auch in solchen Fällen ein einwandfreies Produkt erzeugen. Bevorzugt ist ein pH > 8, besonders bevorzugt 8 - 12, jedoch auch andere pH-Einstellungen ab pH 7 führen zur gewünschten Verbesserung.

Für das einzustellende Rückflußverhältnis gilt bevorzugt, daß bei einem Einsatzprodukt in die Dimethyletherkolonne, bei Zusatz von Waschflüssigkeit einschließlich derselben, das > 0 - 5 Gew.-% Dimethylether enthält, ein Rückflußverhältnis von 1:0,2 bis 1:100, bevorzugt 1:0,2 bis 1:25 gefahren wird, bei einem Gesamt-Einsatzprodukt, das 20 - 80 Gew.-% Dimethylether enthält, ein Rückflußverhältnis von 1:0,2 bis 1:50 gefahren wird, bevorzugt von 1:1 bis 1:5, bei einem Gesamt-Einsatzprodukt, das > 5 - < 20 Gew.-% Dimethylether enthält, ein Rückflußverhältnis von 1:0,2 bis 1:50, bevorzugt von 1:0,2 bis 1:25, und bei einem Gesamt-Einsatzprodukt, das > 80 Gew.-% und < 100 Gew.-% Dimethylether enthält, ein Rückflußverhältnis von 1:0,2 bis 1:50, bevorzugt von 1:0,2 bis 1:10 und besonders bevorzugt von 1:0,2 bis 1:5 gefahren wird, wobei mit zunehmender Dimethyletherkonzentration im Einsatzprodukt im allgemeinen ein abnehmendes Rückflußverhältnis gewählt wird.

Ein Rückflußverhältnis von beispielsweise 1:1 bedeutet, daß die Menge an abgezogenem Dimethylether = 1 Teil ist und die Dampfmenge zur Kondensation am Kopf der Kolonne = 1 + 1 Teile ist, (1. Zahl stellt die abgezogene Menge dar). Ein Rückflußverhältnis von 1:5-8 eignet sich beispielsweise für ein Einsatzprodukt mit einem Dimethylethergehalt von 3-4 Gew.-%. Solche Dimethylethergehalte findet man in Rohmethanol, wie es bei Hochdruck-Methanolsynthesen anfällt. Der Dimethylether kann jedoch nur dann in hochreiner, nahezu quantitativer Menge gewonnen werden, wenn man die erfindungsgemäß genannte Zuführung und die erfindungsgemäßen Abnahmestellen von Dimethylether und Verunreinigungen enthaltendem Destillat wählt. Enthält das Einsatzgemisch beispielsweise 20-80 Gew.-% Dimethylether sowie Methanol und sonstige Komponenten mit Siedepunkten zwischen denen von Methanol und Dimethylether und ggfs. Wasser und sonstige Sauerstoff enthaltende Kohlenwasserstoffe, wie beispielsweise Alkohole mit einer Anzahl C-Atomen > 1, so ist das Rückflußverhältnis auf 1:0,2 bis 1:50, bevorzugt von 1:0,5 bis 1:10 und besonders bevorzugt von 1:1 bis 1:5 einzustellen je nach Dimethylethergehalt. Beispielsweise kann man bei einem Gemisch, das 60 Gew.-% Dimethylether und 15 Gew.-% Methanol enthält, neben Wasser und Verunreinigungen, ein Rückflußverhältnis von 1:1 bis 1:2,5 einstellen.

Solche Einsatzgemische sind typische Gemische, wie sie bei der katalytischen Gewinnung von Dimethylether aus Methanol mit $Al_2O_3$- bzw. $Al_3O_3 \cdot SiO_2$-Katalysatoren im geraden Durchgang am Ausgang des Synthesereaktors anfallen.

Bei Dimethylethergehalten, die zwischen > 5 und < 20 Gew.-% liegen bzw. oberhalb 80 Gew.-% liegen, sind die erfindungsgemäßen Rückflußverhältnisse auf der Basis der angegebenen Werte zu wählen wie z.B. für Gehalte von > 5 - < 20 Gew.-% zwischen einem Rückflußverhältnis von 1:0,2 bis 1:50, bevorzugt von 1:0,2 bis 1:25 zu wählen bzw. bei > 80 bis > 100 Gew.-% auch bei einem Verhältnis 1:0,2 bis 1:10 und besonders bevorzugt von 1:0,2 bis 1:5.

Die angegebenen Rückflußverhältnisse sind jedoch nicht als limitierend anzusehen sondern als Richtwerte, wobei der Fachmann aus den in den Ansprüchen genannten Grenzen ein optimales Rückflußverhältnis wählen kann, wobei in Abhängigkeit von der DME-Konzentration im zu destillierenden Einsatzprodukt das Rückflußverhältnis gewählt wird, das heißt, mit abnehmender DME-Konzentration wird im allgemeinen ein zunehmendes Rückflußverhältnis gewählt.

Die Destillationskolonne zur Gewinnung des reinen Dimethylethers wird im allgemeinen bei einem Druck von 5 - 10 bar betrieben, wobei im Falle eines vorgeschalteten Synthesereaktors bevorzugt eine Anpassung an den Druck im Reaktor erfolgt, wobei im allgemeinen die Destillation bei einem etwas niedrigeren Druck als die Synthese betrieben wird. Druck außerhalb des Bereichs von 5 - 10 bar, insbesondere bei höherem Druck sind erfindungsgemäß ebenfalls möglich. Der Durchsatz wird wie üblich, durch die Auslegung der Kolonne, die Wärmezufuhr und das Rückflußverhältnis bestimmt.

Zur nahezu quantitativen Gewinnung des reinen Dimethylethers, insbesondere bei kleinen Gehalten des Dimethylethers im Einsatzprodukt kann erfindungsgemäß das über Kopf der Kolonne gehende Abgasgemisch, das im allgemeinen $CO_2$, $N_2$, Kohlenwasserstoffe und noch kleine Anteile an Dimethylether enthält, gewaschen werden. Die den Dimethylether enthaltende Waschflüssigkeit kann in die Kolonne oder in den Dimethylether-Synthesereaktor rückgeführt werden.

Geeignete Waschflüssigkeiten sind beispielsweise Methanol oder Sumpfprodukte der Dimethylether-Destillationskolonne. Das Waschen kann im Gleich-, Kreuz- oder Gegenstrom erfolgen, bevorzugt jedoch im Gegenstrom.

Ferner kann das die Verunreinigungen enthaltende Destillat zusätzlich in einem Seitenstripper gestrippt werden, wobei das Stripp-Produkt wieder in die Kolonne zurückgeführt wird. Hierdurch kann ggfs. an dieser Stelle ausgetretener Dimethylether nahezu vollständig rückgeführt werden.

Wie für den Fachmann deutlich ist, wird erfindungsgemäß das Methanol von Wasser in einer 2. Kolonne abgetrennt.

Dieses Methanol kann in den DME-Synthesereaktor rückgeführt werden, es kann jedoch auch anderen bekannten Methanolverwendungen zugeführt werden.

Nach dem erfindungsgemäßen Verfahren können Destillationskolonnen nach dem Stand der Technik entsprechend der Anlagenkapazität eingesetzt werden.

Die Böden können die dem Stand der Technik entsprechenden Böden sein, wie beispielsweise Ventilböden, Siebböden, Glockenböden mit und ohne Kamin u.a.

Grundsätzlich können auch Füllkörper oder Packungen wie beispielsweise keramische, metallische oder Kunststoff-Materialien, Glasmaterialien, Packungen aus Draht und dergleichen als Füllung für die Destillationskolonne verwendet werden, wobei die erfindungsgemäße Einsatzproduktzuführung, bzw. die Entnahmestellen für Dimethylether und Vorlauf gemäß den erforderlichen Bodenzahlen errechnet werden können auf der Grundlage der in der vorliegenden Erfindung angegebenen praktischen Böden.

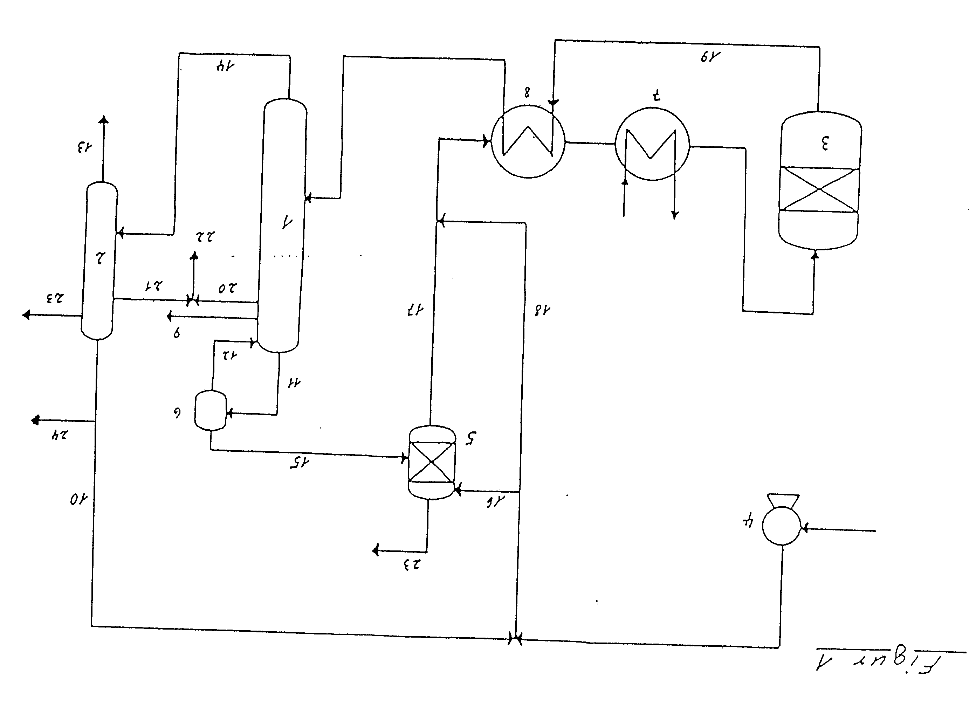

Die Erfindung wird beispielhaft mit Hilfe der Figuren 1 bis 3 näher erläutert.

In Figur 1 stellt (3) den Dehydratisierungsreaktor dar. Über Pumpe (4), Leitung (18) und Wärmetauscher (8) und (7) wird frisches Methanol dem Reaktor zugeführt. (1) stellt die Destillationskolonne zur Gewinnung des reinen Dimethylethers dar. Die Kolonne wird im allgemeinen in einem Druckbereich betrieben, der dem Druck in Synthesereaktor (3) angepaßt ist, üblicherweise bei etwas niedrigerem Druck. Liegt beispielsweise der Synthesedruck bei 8 - 12 bar, so kann die Kolonne beispielsweise bei 6 - 11,5 bar betrieben werden. Diese Zahlen sind jedoch nicht als einschränkend anzusehen.

Im allgemeinen wird jedoch bevorzugt bei einer Druckdifferenz von 0 bis zu 10 bar gearbeitet. Der Dimethylether wird bei (9) in hochreiner Form abgezogen. Kopfgas gelangt über (11) in Kondensator (6). Rückfluß fließt über (12) wieder auf Kolonne (1). Das Abgas fließt über (15) in Wäscher (5), in dem es mit Methanol (16) gewaschen wird. Grundsätzlich können auch andere Waschflüssigkeiten eingesetzt werden, wie beispielsweise Rohmethanol oder Sumpf der Kolonne 1, wobei im letzteren Fall die den Dimethylether enthaltende Waschflüssigkeit wieder der Kolonne 1 zugeführt werden kann. Kleine Mengen Dimethylether gelangen über (17) zusammen mit Waschmethanol in den Synthesereaktor (3). Das Abgas entweicht bei (23).

Das Syntheseprodukt (19) fließt über (8) nach (1). Bei (20) werden die zwischen Methanol und Dimethylether siedenden Verunreinigungen abgezogen und gelangen über (22) beispielsweise zur Verbrennung. Über die beispielhaften Zuführungen (24), (25) und/oder (26) kann erfindungsgemäß Waschflüssigkeit zugeführt werden.

Über (14) fließt der im wesentlichen Methanol und Wasser enthaltende Sumpf in Methanolkolonne (2). Diese wird im allgemeinen bei Normaldruck betrieben, kann jedoch prinzipiell auch bei etwas erhöhtem oder etwas niedrigerem Druck betrieben werden, jedoch im allgemeinen unterhalb des Druckes der Kolonne (1). Über (10) wird Methanol abgezogen und zum Synthesereaktor zurückgeführt. Aus dem Sumpf wird über (13) Abwasser abgezogen. Über (21) können im Bedarfsfall Verunreinigungen entnommen werden mit höheren Siedepunkten als dem des Methanols. Das Methanol kann auch an einem der oberen Böden der Kolonne (2) als Seitenabzug entnommen werden (23). Kleine Mengen an Verunreinigungen können dann noch über Kopf entnommen werden (24).

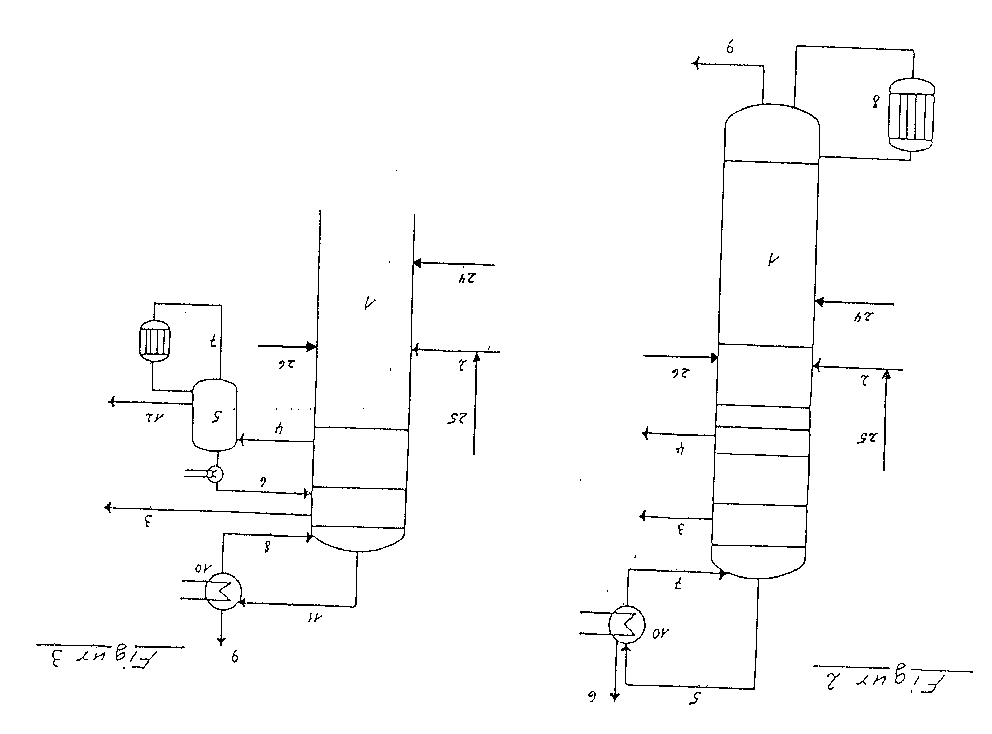

In Figur 2 stellt (1) die Destillationskolonne zur Gewinnung von reinem Dimethylether dar. Das Einsatzprodukt wird über (2) zugeführt. Der reine Dimethylether wird bei (3) entnommen. Die Verunreinigungen werden bei (4) abgezogen. Das Kopfprodukt fließt über (5) zum Kondensator (10). Rückfluß fließt über (7) zu Kolonne (1). Abgas entweicht bei (6). (8) stellt den Reboiler-Kreislauf dar. Bei (9) wird der Sumpf abgezogen. Über (24), (25) und (26) kann erfindungsgemäß Waschflüssigkeit zugeführt werden.

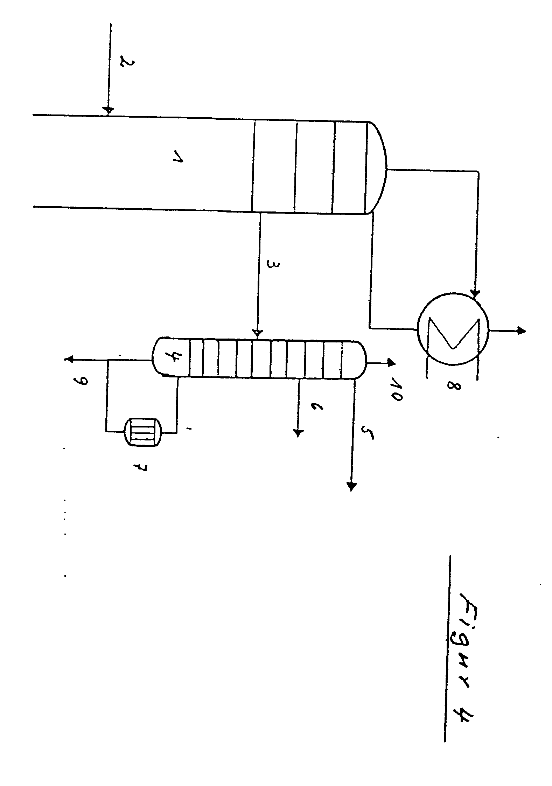

In Figur 3 fließt das die Verunreinigungen enthaltende Destillat über (4) zum Stripper (5), der mit Reboiler-Kreislauf (7) ausgerüstet ist. Über (24), (25) und (26) kann erfindungsgemäß Waschflüssigkeit zugeführt werden.

Abgestrippter Dimethylether fließt über (6) zur Kolonne (1) zurück.

Bei (2) wird Einsatzprodukt zugeführt. Bei (3) wird reiner Dimethylether entnommen, Kopfgas gelangt über (11) in Kondensator (10). Rückfluß fließt über (8) zur Kolonne (1). Abgas entweicht bei (9). Bei (12) werden die von Dimethylether befreiten Verunreinigungen abgenommen.

Die Waschflüssigkeit kann auch in Dampfform oder teilweise in Dampfform und teilweise flüssig in die Kolonne eingeführt werden.

In Figur 4 fließt ein praktisch 100 %iger Dimethylether mit schwacher Geruchsverunreinigung von Kolonne (1) über Leitung (3) in eine 2. Dimethyletherkolonne (4). Dort wird erfindungsgemäß über (5) reiner, geruchsfreier DME abgezogen. Eine kleine Menge an Geruchskomponenten enthaltendem Destillat wird über (6) abgenommen. (7) stellt den Reboiler dar, (9) den Sumpfabzug und (10) den Kopfabzug.

Gemäß vorliegender Erfindung wird aus der Destillationskolonne der hochreine Dimethylether praktisch quantitativ gewonnen. Der Dimethylether ist geruchsfrei, enthält weniger als 10 ppm Methanol, maximal 0,1 Gewichtsprozent Kohlenwasserstoffe und besitzt eine Reinheit von wenigstens 99,9 Gew.-% Dimethylether. Der erfindungsgemäß gewonnene Dimethylether ist für jede Anwendung im Aerosolbereich hervorragend geeignet.

In den folgenden Beispielen wird die Erfindung näher erläutert, wobei in den Beispielen 1 - 7 ein die Verunreinigungen enthaltendes Destillat vor Einsatz in die Dimethyletherkolonne einem Dehydratisierungsprodukt zugemischt wurde. Dieses Destillat war aus einer in Betrieb befindlichen Dimethyletherkolonne unterhalb des DME-Abzugsbodens erhalten worden. Es bestand jeweils überwiegend aus Dimethylether. Zusätzlich wurden kleine Mengen an höher siedenden Verunreinigungen aus einer Methanoldestillation hinzugefügt.

(Die Angabe "Geruch" bezieht sich auf den durch Beimengungen verursachten Geruch, nicht jedoch auf den sehr leichten Eigengeruch des DME. "Geruchsfrei" bedeutet demgemäß, daß kein durch Beimengungen bzw. Verunreinigungen hervorgerufener Geruch festzustellen war.)

Die angegebenen ppm-Mengen an Methanol und Kohlenwasserstoffen wurden in der Gesamtbilanz nicht berücksichtigt.

## Beispiel 1

Einer Kolonne mit 65 Ventilböden wurden am 48. Boden (vom Kopf der Kolonne) 4493 kg/h eines Dehydratisierungsproduktes zuge führt, das aus 2600 kg Dimethylether (DME), 720 kg Methanol, 1060 kg Wasser und 113 kg Verunreinigungen (hiervon 90 kg DME) bestand sowie 2000 kg/h Reinmethanol über die gleiche Zuführungsleitung.

Die Kolonne wurde bei 8 bar betrieben.

Das Rückflußverhältnis lag bei 1:3.

Am Kopf der Kolonne wurden ca. 5 kg/h Abgas abgezogen, das im wesentlichen aus Inertgas bestand.

Am 5. Boden der Kolonne (vom Kolonnenkopf) wurden 2609 kg/h reiner, geruchsfreier Dimethylether abgezogen mit einem Methanolgehalt von 1 ppm und einem Gehalt an Kohlenwasserstoffen unterhalb der Nachweisgrenze (< 50 ppm).

Am 37. Boden (vom Kolonnenkopf) wurden 92 kg/h (81 kg hiervon DME) an Verunreinigungen abgezogen. 12 kg/h höher siedende Komponenten (als Methanol), 2720 kg/h Methanol und 1060 kg/h Wasser wurden am Sumpf abgezogen und einer Kolonne zur Methanoldestillation zugeführt.

Vergleichsbeispiel 1a

Beispiel 1 wurde wiederholt, jedoch ohne Zuführung von zusätzlichem Methanol. Das Rückflußverhältnis betrug 1:3.

Es wurden 6 kg/h Gase am Kopf der Kolonne abgezogen, die im wesentlichen aus Inertgas bestanden.

Am 5. Boden wurden 2594 kg/h eines reinen, geruchsfreien Dimethylethers abgezogen mit einem Methanolgehalt von 1 ppm und einem Kohlenwasserstoffgehalt unterhalb der Nachweisgrenze.

Am 37. Boden wurden 107 kg/h (96 kg hiervon DME) an Verunreinigungen abgezogen. 12 kg/h an höher siedenden Verunreinigungen, 720 kg/h Methanol und 1060 kg/h Wasser wurden am Sumpf abgezogen.

Beispiel 2

In die gleiche Kolonne wie in Beispiel 1 wurde das gleiche Einsatzprodukt eingesetzt.

Die Kolonne wurde wie in Beispiel 1 betrieben, jedoch wurden die Verunreinigungen am 7. Boden (vom Kolonnenkopf) abgezogen. Der erhaltene reine Dimethylether war nahezu geruchsfrei und enthielt <1ppm Methanol und einen Gehalt an Kohlenwasserstoffen unterhalb der Nachweisgrenze.

Vergleichsbeispiel 2a

Beispiel 2 wurde wiederholt, jedoch ohne Zuführung von zusätzlichem Methanol.

Der erhaltene Dimethylether hatte einen deutlich stärkeren Geruch als in Beispiel 2, enthielt <1 ppm Methanol und 55 ppm Kohlenwasserstoffe.

Beispiel 3

Einer Destillationskolonne mit 100 Ventilböden wurden pro Stunde 66.000 kg eines Dehydratisierungsgemischs zugeführt, das aus 62.000 kg Methanol, 2500 kg Dimethylether, 980 kg Wasser und 520 kg (hiervon 237 kg DME) Verunreinigungen bestand.

Die Kolonne wurde bei 7,5 bar betrieben.

Das Rückflußverhältnis betrug 1:10.

Zusätzlich wurden 10.000 kg/h Rohmethanol am 60. Boden (vom Kolonnenkopf) zugeführt.

Das Rohmethanol stammte aus einer Lurgi-Niederdruckmethanolanlage und enthielt 93 Gew.-% Methanol, 0,5 Gew.-% Kohlenwasserstoffe + höhere Alkohole (0,3 %) und 6,5 Gew.-% Wasser.

Das Dehydratisierungsprodukt wurde am 48. Boden (vom Kolonnenkopf) zugeführt.

298 kg/h (hiervon 209 kg DME) Verunreinigungen wurden am 42. Boden (vom Kopf der Kolonne) abgezogen.

2528 kg/h reiner Dimethylether wurden am 4. Boden (vom Kolonnenkopf) abgezogen.

Über Kopf wurden 11 kg/h Abgas abgezogen, das überwiegend aus Inerten bestand.

Aus dem Sumpf wurden 244 kg/h höher siedende Verunreinigungen (50 kg aus dem Rohmethanol), 71.300 kg/h Methanol und 1630 kg/h Wasser abgezogen.

Der Dimethylether enthielt < 1 ppm Methanol, 90 ppm Kohlenwasserstoff und war völlig geruchlos.

Vergleichsbeispiel 3a

Beispiel 3 wurde wiederholt, jedoch ohne Rohmethanolzufuhr.

Das Rückflußverhältnis betrug 1:7.

Es wurden 2487 kg/h geruchloser Dimethylether erhalten, der Kohlenwasserstoffe < 50 ppm und < 1 ppm Methanol enthielt.

Beispiel 4

In eine Glockenbodenkolonne mit 100 Böden wurde am 46. Boden ein Gemisch von 40.000 kg/h Rohmethanol und 3500 kg/h Dehydratisierungsprodukt eingesetzt.

Das Rohmethanol entsprach der in Beispiel 3 eingesetzten Qualität und wurde direkt aus dem Druckabscheider der Methanolsynthese entnommen.

Das Dehydratisierungsprodukt bestand aus 2200 kg Dimethylether, 861 kg Wasser, 385 kg Methanol und 54 kg (mit 29 kg DME) Verunreinigungen. Es wurde bei 6,5 bar und einem Rückflußverhältnis von 1:12 gefahren.

Am 6. Boden der Kolonne (vom Kopf der Kolonne) wurden 2183 kg/h reiner Dimethylether abgezogen. Über Kopf gingen 8 kg/h Abgas.

Am 36. Boden wurden 55 kg/h (mit 46 kg DME) Verunreinigungen abgezogen. Am Sumpf wurden 3461 kg/h Wasser, 37585 kg/h Methanol und 216 kg/h an höher siedenden Verunreinigungen abgezogen. Es wurde ein völlig geruchsfreier Dimethylether erhalten, der < 10 ppm Kohlenwasserstoffe und 1 ppm Methanol enthielt.

## Beispiel 5

Beispiel 1 wurde wiederholt, jedoch wurden anstelle von Rohmethanol 20.000 kg/h Wasser zugesetzt.
Es wurde ein reiner, geruchsfreier Dimethylether erhalten, der 1 ppm Methanol und < 50 ppm Kohlenwasserstoffe enthielt.
Ähnliche Ergebnisse wurden mit Methanol/Wassergemischen erhalten.

## Beispiel 6

Die Aufarbeitung des Dehydratisierungsproduktes (3000 kg/h Einsatzprodukt mit 65 Gew.-% Dimethylether) erfolgt ein 2 hintereinander geschalteten kontinuierlich arbeitenden Destillationskolonnen, von denen die erste mit 25 Ventilböden und die 2. mit Raschigringen ausgerüstet war. Der reine Dimethylether wurde in der ersten Kolonne bei 7 bar am 4. Boden (vom Kopf der Kolonne) bei einem Rückflußverhältnis von 1:2 gewonnen. Das Einsatzprodukt wurde am 12. Boden (vom Kopf der Kolonne) zugeführt. Die Verunreinigungen wurden am 14. Boden abgezogen. In der 2. Kolonne wurde nicht umgesetztes Methanol zurückgewonnen.
Es wurde ein geruchsfreier Dimethylether erhalten.

## Vergleichsbeispiel 6a

Es wurde wie in Beispiel 6 gearbeitet, jedoch die Verunreinigungen am Kopf der 2. Kolonne abgezogen.
Der so gewonnene Dimethylether hatte einen deutlichen durch Verunreinigungen bedingten Geruch.

## Vergleichsbeispiel 6b

Es wurde wie in Beispiel 6a gearbeitet, zusätzlich wurden jedoch 3000 kg/h Reinmethanol gemeinsam mit dem Dehydratisierungsprodukt in die Kolonne eingesetzt.
Der Dimethylether besaß einen schwachen Geruch.

## Beispiel 7

Gemäß Beispiel 3 wurden 65.955 kg/h Dehydratisierungsprodukt eingesetzt, welches 7.200 kg DME, 57.230 kg Methanol und 1.050 kg Wasser und 475 kg (hiervon 425 kg DME) Verunreinigungen enthielt.
Es wurde bei einem Rückflußverhältnis von 1:0,2 gearbeitet.
Die Menge an am 12. Boden abgezogenem Dimethylether betrug nur 7342 kg.
Obgleich am 18. Boden ein die Verunreinigungen enthaltendes Destillat abgenommen wurde, welches 283 kg Dimethylether enthielt (abgezogene Menge insgesamt: 333 kg) wobei über Kopf 3 kg/h Abgas abgenommen wurden, besaß der Dimethylether einen schwachen Geruch aufgrund des geringen Rückflußverhältnisses bezogen auf die geringe DME-Konzentration im Einsatzprodukt.

## Vergleichsbeispiel 7a

Beispiel 7 wurde wiederholt, jedoch so viel NaOH in Wasser gelöst (ca. 12 %ig), zugegeben, daß der pH im Sumpf zwischen 10 und 11 lag.
Der so erhaltene Dimethylether war völlig geruchsfrei.

## Beispiel 8

4000 kg Dehydratisierungsprodukt, das 3120 kg Dimethylether, 420 kg Wasser 46 kg (40 kg hiervon waren DME) an Verunreinigungen, sowie 414 kg Methanol enthielt, wurde in einer Kolonne mit 50 Ventilböden destilliert (Feedboden: 27. Boden).
Die Kolonne wurde bei 8 bar und einem Rückflußverhältnis von 1:2,5 betrieben.
3150 kg Dimethylether wurden am 15. Boden (von oben) abgenommen. Am 18. Boden wurden 16 kg (hiervon 10 kg DME) an Verunreinigungen enthaltendem Destillat abgenommen.
Über Kopf wurden 2 kg im wesentlichen aus Inerten bestehendem Abgas abgezogen.
Zur Methanolkolonne wurde ein Gemisch von 420 kg Wasser und 414 kg Methanol gefahren. (Jeweils pro Stunde).
Verursacht durch die nur geringe Menge an abgezogenem, die Verunreinigungen enthaltendem Destillat, wobei der Abzugsboden für dasselbe nur 3 Böden unter dem DME-Abzug lag, und die hohe Ausbeute an abdestilliertem DME (99,6 % des DME im Einsatzprodukt) am 15. Boden besaß der Dimethylether einen schwachen Geruch.

## Vergleichsbeispiel 8a

Beispiel 8 wurde wiederholt, jedoch soviel wässrige KOH hinzugefügt (ca. 20 %ig), daß der pH bei 8 - 9 im Sumpf der Kolonne lag.
Der so erhaltene Dimethylether war völlig geruchsfrei.

Ein analoges Ergebnis wurde durch Zusatz von Ca-(OH)$_2$ erhalten.

Die Einsatzprodukte in die Dimethyletherkolonne wurden im Falle der folgenden Beispiele 9 - 10 kontinuierlich aus einem Synthesereaktor in die Kolonne eingesetzt. Die Gesamtlaufzeiten der Versuche lagen bei jeweils 720 Stunden.

Beispiel 9

Das Reaktorausgangsprodukt bestand aus 1046 kg nicht umgesetztem Methanol, 1442 kg Reaktionswasser und 3773 kg Dimethylether.

Dieses Gemisch gelangte mit einer Temperatur von 112 °C in die Destillationskolonne am 53. Boden.

Die Kolonne hatte eine Gesamtbodenzahl von 61 Ventilböden. Das Rückflußverhältnis lag bei 1:3.

Am 50. Boden wurden 60 kg/h an Verunreinigungen abgezogen. (57 kg dieser Fraktion bestanden aus Dimethylether.)

Am 45. Boden wurden 3716 kg reiner Dimethylether abgezogen.

3 kg an Gasen, die überwiegend aus Inerten bestanden gingen über Kopf der Kolonne.

Der Sumpf bestand aus 2486 kg und wurde in die mit 40 Ventilböden ausgerüstete Methanolkolonne eingesetzt.

Dort erfolgte eine quantitative Trennung in 1044 kg Methanol, das über Kopf abgenommen wurde und 1442 kg Wasser, das aus dem Sumpf abgezogen wurde. (Jeweils pro Stunde).

Der Dimethylether war geruchsfrei. Der Methanolgehalt lag bei 2 ppm, der Kohlenwasserstoffgehalt bei < 50 ppm.

Beispiel 10

Beispiel 10 wurde entsprechend Beispiel 9 durchgeführt.

Das Einsatzprodukt in die Dimethyletherkolonne bestand aus 5979 kg nicht umgesetztem Methanol, 79 kg Reaktionswasser und 203 kg Dimethylether. (Jeweils pro Stunde).

Das Gemisch wurde am 45. Boden in die Dimethyletherkolonne eingesetzt.

Das Rückflußverhältnis lag bei 1:15.

Am 50. Boden wurden 21 kg/h an Verunreinigungen abgezogen. (Hiervon bestanden 10 kg aus Dimethylether).

Am 33. Boden vom Kopf der Kolonne wurden 193 kg reiner Dimethylether abgezogen. Am Kopf der Kolonne fiel 1 kg Inerte/h an.

6047 kg Sumpfprodukt wurden in die Methanolkolonne eingesetzt und dort in 5978 kg Methanol und 79 kg Wasser getrennt.

Der Dimethylether war geruchsfrei.

Der Methanolgehalt lag bei 3 ppm, der Kohlenwasserstoffgehalt bei < 50 ppm.

In den Beispielen 11 - 13 wurden unterschiedlich zusammengesetzte Gemische eingesetzt (Laufzeiten jeweils 48 Stunden).

Beispiel 11

Beispiel 11 wurde entsprechend Beispiel 9 durchgeführt.

Das Reaktionsprodukt bestand aus 188 kg nicht umgesetztem Methanol, 1708 kg Reaktionswasser und 4365 kg Dimethylether.

Das Gemisch wurde am 28. Boden in die Dimethyletherkolonne eingesetzt.

Das Rückflußverhältnis lag bei 1:0,4.

Am 22. Boden wurden 91 kg/h an Verunreinigungen abgezogen. Hiervon bestanden 90 kg aus Dimethylether.

Am 17. Boden wurden 4275 kg reiner Dimethylether abgezogen.

Am Kolonnenkopf fielen 4 kg Inerte an. 1895 kg Sumpfprodukt wurden in die Methanolkolonne eingesetzt und dort in 187 kg Methanol und 1708 kg Reaktionswasser getrennt.(Jeweils pro Std.)

Das Methanol wurde in die Dimethylether-Synthese rückgeführt.

Der Dimethylether besaß einen sehr schwachen Geruch.

Vergleichsbeispiel 11a

Beispiel 11 wurde wiederholt, jedoch der Sumpf der Dimethyletherkolonne auf pH 7,5 - 8,5 eingestellt (mit 10 %iger NaOH-Lösung).

Der so gewonnene Dimethylether war ohne Geruch.

Das gleiche Ergebnis wurde mit LiOH und Mg(OH)$_2$ als Base erhalten.

Beispiel 12

In eine mit 65 Glockenböden ausgestattete Kolonne wurden 7185 kg/h eines Dehydratisierungsgemischs aus dem DME-Synthesereaktor am 51. Boden eingesetzt, das aus 4300 kg DME, 1200 kg Methanol und 1685 kg Wasser bestand.

Am 7. Boden wurden 4220 kg DME abgezogen.

Am 40. Boden wurden 82 kg Verunreinigungen (hiervon 80 kg DME) enthaltendes Destillat abgezogen.

In die Methanolkolonne gelangten 1198 kg Methanol und 1685 kg Wasser.

Das Methanol wurde abdestilliert und in den Synthesereaktor rückgeführt.

Der Dimethylether hatte einen schwachen Geruch.

Vergleichsbeispiel 12 a

Beispiel 12 wurde wiederholt, jedoch der Sumpf der Dimethyletherkolonne auf pH 12 (mit NaOh 20 %ig) eingestellt.

Der so gewonnene DME war völlig geruchsfrei.

Beispiel 13

In eine Kolonne mit 10 Ventilböden wurden 3.000 kg/h eines Einsatzproduktes eingesetzt, das aus 2940 kg Dimethylether, 30 kg Methanol und 30 kg $H_2O$ bestand.

Das Gemisch wurde am 1. Boden über dem Kolonnensumpf zugeführt. Am 5. Boden (von unten) wurden 1465 kg Geruchskomponenten enthaltendes DME abgezogen (technische Qualität). Am 9. Boden von unten wurden 1475 reiner, geruchsfreier Dimethylether abgezogen mit 1 ppm Methanol und < 50 ppm Kohlenwasserstoffen.

Beispiel 1 und 2 zeigen, daß sich durch zusätzliches Methanol die Menge an gewinnbarem reinen Dimethylether erhöht.

Aus Beispiel 3 und Vergleichsbeispiel 3a geht hervor, daß auch bei Einsatz von Rohmethanol als Waschflüssigkeit eine erhöhte Menge an reinem Dimethylether gewonnen werden kann.

In Beispiel 4 ist eine typische integrierte Destilation von Rohmethanol aus einer Methanolsyntheseanlage und Dehydratisierungsprodukt aus einer Dimethylether-Syntheseanlage offenbart. Das Verhältnis der beiden Produkte ist ca. 11:1, es kann jedoch, wie oben ausgeführt, in Abhängigkeit von der Kapazität der Anlagen stark variieren.

Das Beispiel zeigt, daß ein hochreiner Dimethylether bei einer solchen Fahrweise gewonnen werden kann, ohne daß die Gewinnung eines spezifikationsgerechten Reinmethanols beeinträchtigt wird (im Beispiel nicht offenbart).

Aus Beispiel 5 geht hervor, daß auch mit Wasser und Methanol/Wasser-Gemischen die Qualität des Dimethylethers verbessert werden kann.

Schließlich zeigen die Beispiele 6 und die Vergleichsbeispiele 6a und 6b, daß auch bei Abweichung von der erfindungsgemäßen Destillationsmethode, die Verwendung von Waschflüssigkeit die Qualität des Dimethylethers verbessert.

Die Beispiele 7 und 8 zeigen, daß sich für den Fall, daß bei sehr geringem Rückflußverhältnis bezogen auf die DME-Konzentration im Einsatzprodukt und Abzug von Verunreinigungen und reinem Dimethylether im Grenzbereich der beanspruchten Abzugsböden ein auftretender Geruch durch Zugabe von einer Base bzw. Einstellen eines pH's oberhalb 7 beseitigt werden kann.

Aus den Beispielen 9 und 10 geht hervor, daß bei einem Abstand von mindestens 8 Böden zwischen Zuführungsboden und Dimethyletherabzug ein geruchsfreier Dimethylether erhalten wird.

Die Beispiele 11 und 12 zeigen, daß durch Recycling des in der Methanolkolonne abdestillierten Methanols zum Dimethylether-Synthesereaktor ein schwacher Geruch im Dimethylether auftreten kann, der sich jedoch durch die erfindungsgemäße pH-Einstellung beseitigen läßt.

Beispiel 13 zeigt, daß auch in einer sehr kurzen Destillationskolonne erfindungsgemäß reiner, geruchsfreier Dimethylether gewonnen werden kann.

Die vorliegende Erfindung zeigt, daß grundsätzlich bei allen erfindungsgemäßen Fahrweisen, sowie Fahrweisen im Grenzbereich der Schutzansprüche, die Einstellung des pH's von großer Bedeutung bezüglich der Sicherung einer einwandfreien DME-Produktion ist.

**Ansprüche**

1. Verfahren zur Gewinnung von reinem Dimethylether durch katalytische Dehydratisierung von Methanol bei einer Temperatur von 140 - 500 °C und einem Druck von 1 - 50 bar und destillative Aufarbeitung des Dehydratisierungsprodukts, durch Einsatz des gasförmigen und/oder flüssigen Dehydratisierungsprodukts in die Kolonne zur Gewinnung von reinem Dimethylether mindestens 1 Boden, bevorzugt mindestens 5 Böden und besonders bevorzugt mindestens 10 Böden oberhalb des Kolonnensumpfes, dadurch gekennzeichnet

a, daß (eine) Verunreinigungen enthaltende flüssige und/oder gasförmige Fraktion(en), an einem oder mehreren Böden abgezogen wird (werden), wobei

die Verunreinigungen enthaltende(n) Fraktion(en) um mindestens 3, bevorzugt mindestens 5 Böden unterhalb des untersten Abzugsbodens für reinen Dimethylether abgezogen wird (werden) bis mindestens 5 Böden bevorzugt mindestens 10 Böden oberhalb des Sumpfes.

b, daß der Boden, oder im Falle mehrerer Abzugsböden der unterste Boden an dem der reine Dimethylether abgezogen wird

bei nur einem Zuführungsboden um mindestens 8 Böden, bevorzugt mindestens 10 Böden und besonders bevorzugt um mindestens 12 Böden oberhalb des Bodens liegt, an dem das Einsatzprodukt zugeführt wird

bei mehreren Zuführungsböden bei einer am obersten Zuführungsboden zugeführten Menge von > 0 - < 100 % der Gesamtmenge, um mindestens 1 Boden bis mindestens 8 Böden oberhalb des obersten Zuführungsbodens liegt, wobei mit steigender zugeführter Menge der Bodenabstand zunimmt.

2. Verfahren nach Anspruch 1, dadurch gekennzeichnet, daß zur destillativen Aufarbeitung des Dehydratisierungsprodukts zu reinem Dimethylether im Sumpf der Dimethyletherkolonne ein pH von mindestens 7, bevorzugt > 7 eingestellt wird.

3. Verfahren nach den Ansprüchen 1 und 2, dadurch gekennzeichnet, daß zur pH-Einstellung NaOH und/oder KOH zugesetzt wird.

4. Verfahren nach den Ansprüchen 1 - 3, dadurch gekennzeichnet, daß zusätzlich zum Dehydratisierungsprodukt aus dem Dimethylether-Synthesereaktor. eine Waschflüssigkeit eingesetzt wird.

5. Verfahren nach den Ansprüchen 1 - 4, dadurch gekennzeichnet, daß die Waschflüssigkeit Methanol oder ein Methanol/Wassergemisch ist.

6. Verfahren nach den Ansprüchen 1 - 4, dadurch gekennzeichnet, daß die Waschflüssigkeit Wasser ist.

7. Verfahren nach den Ansprüchen 1 - 6, dadurch gekennzeichnet, daß die am Kopf der Destillationskolonne zur Gewinnung reinen Dimethylethers abgezogenen leichten Komponenten, die allgemein $CO_2$, $N_2$, leichte Kohlenwasserstoffe und Dimethylether enthalten, in Gegen-, Kreuz- oder Gleichstrom zur Gewinnung des Dimethylethers gewaschen werden, vorzugsweise mit Methanol.

8. Verfahren nach den Ansprüchen 1 - 7, dadurch gekennzeichnet, daß die Waschflüssigkeit in die Destillationskolonne unter halb des Abzugsbodens für das die Verunreinigungen enthaltende Destillat eingesetzt wird.

9. Verfahren nach den Ansprüchen 1 - 8, dadurch gekennzeichnet, daß bei einem Dehydratisierungsprodukt in die Dimethyletherkolonne, bei Zusatz von Waschflüssigkeit einschließlich derselben, das > 0 - 5 Gew.-% Dimethylether enthält, ein Rückflußverhältnis von 1:0,2 bis 1:100, bevorzugt 1:0,2 bis 1:25 gefahren wird, bei einem Gesamt-Einsatzprodukt, das 20 - 80 Gew.-% Dimethylether enthält, ein Rückflußverhältnis von 1:0,2 bis 1:50 gefahren wird, bevorzugt von 1:1 bis 1:5, bei einem Gesamt-Einsatzprodukt, das > 5 -< 20 Gew.-% Dimethylether enthält, ein Rückflußverhältnis von 1:0,2 bis 1:50, bevorzugt von 1:0,2 bis 1:25, und bei einem Gesamt-Einsatzprodukt, das > 80 Gew.-% und < 100 Gew.-% Dimethylether enthält, ein Rückflußverhältnis von 1:0,2 bis 1:10, bevorzugt von 1:0,2 bis 1:5 gefahren wird, wobei mit zunehmender Dimethyletherkonzentration im Einsatzprodukt im allgemeinen ein abnehmendes Rückflußverhältnis gewählt wird.

Figur 1

Figur 2

Figur 3

Figur 4